(19) Europäisches Patentamt / European Patent Office / Office européen des brevets

(11) **EP 4 572 655 B1**

(12) **EUROPEAN PATENT SPECIFICATION**

(45) Date of publication and mention of the grant of the patent:
**29.07.2026 Bulletin 2026/31**

(21) Application number: **22958967.6**

(22) Date of filing: **22.09.2022**

(51) International Patent Classification (IPC):
**G06F 3/01** *(2006.01)*   **A61B 3/14** *(2006.01)*
**A61B 3/15** *(2006.01)*   **A61B 5/11** *(2006.01)*
**A61B 5/16** *(2006.01)*

(52) Cooperative Patent Classification (CPC):
**A61B 5/163; A61B 5/1114; A61B 5/1128;
G02B 27/0093; G06F 3/012; G06F 3/013;**
A61B 5/18

(86) International application number:
**PCT/CA2022/051410**

(87) International publication number:
**WO 2024/059927 (28.03.2024 Gazette 2024/13)**

(54) **METHODS AND SYSTEMS FOR GAZE TRACKING USING ONE CORNEAL REFLECTION**

VERFAHREN UND SYSTEME ZUR BLICKVERFOLGUNG UNTER VERWENDUNG EINER HORNHAUTREFLEXION

PROCÉDÉS ET SYSTÈMES DE SUIVI DU REGARD À L'AIDE D'UNE RÉFLEXION CORNÉENNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**25.06.2025 Bulletin 2025/26**

(73) Proprietor: **Huawei Technologies Co., Ltd.
Shenzhen, Guangdong 518129 (CN)**

(72) Inventors:
- **CHUGH, Soumil
  Markham, Ontario L3R 5A4 (CA)**
- **YE, Juntao
  Markham, Ontario L3R 5A4 (CA)**
- **EIZENMAN, Moshe
  Toronto, Ontario M3H 5P3 (CA)**

(74) Representative: **Maiwald GmbH
Engineering
Elisenhof
Elisenstrasse 3
80335 München (DE)**

(56) References cited:
US-A1- 2011 141 010    US-A1- 2016 262 614
US-A1- 2019 213 402    US-A1- 2022 050 521

- OHNO ET AL.: "FreeGaze: A Gaze Tracking System for Everyday Gaze Interaction", ASSOCIATION FOR COMPUTING MACHINERY, PROCEEDINGS OF THE 2002 SYMPOSIUM ON EYE TRACKING RESEARCH & APPLICATIONS, 25 March 2002 (2002-03-25), pages 125 - 132, XP002336136, Retrieved from the Internet <URL:https://dl.acm.org/doi/abs/10.1145/507072.507098> [retrieved on 20221103], DOI: 10.1145/507072.507098
- GUESTRIN ET AL.: "General Theory of Remote Gaze Estimation Using the Pupil Center and Corneal Reflections", IEEE TRANSACTIONS ON BIOMEDICAL ENGINEERING, vol. 53, no. 6, 5 June 2006 (2006-06-05), pages 1124 - 1133, XP002521985, Retrieved from the Internet <URL:https://ieeexplore.ieee.org/document/1634506> [retrieved on 20221103], DOI: 10.1109/TBME.2005.863952

EP 4 572 655 B1

- **NIU ET AL.: "Real-Time Localization and Matching of Corneal Reflections for Eye Gaze Estimation via a Lightweight Network", ASSOCIATION FOR COMPUTING MACHINERY, CHINESE CHI 2021: THE NINTH INTERNATIONAL SYMPOSIUM OF CHINESE CHI, 16 October 2021 (2021-10-16), pages 33 - 40, XP058899055, Retrieved from the Internet <URL:https://dl.acm.org/doi/abs/10.1145/3490355.3490359> [retrieved on 20221103], DOI: 10.1145/3490355.3490359**

**Description**

TECHNICAL FIELD

The present disclosure relates to the field of computer vision, in particular methods and devices for estimating gaze direction.

BACKGROUND

Gaze tracking is a useful indicator of human visual attention and has wide ranging applications in areas such as human-computer interaction, automotive safety, medical diagnoses, and accessibility interfaces, among others. An eye-tracking or gaze estimation system tracks eye movements and estimates a point of gaze either on a display screen or in the surrounding environment. To capitalize on the benefits of gaze tracking, monitoring systems should preferably operate with a high degree of accuracy, be robust to head movements and be minimally affected by image noise.

A common approach for gaze estimation is video-based eye-tracking. In such systems, a camera is used to capture eye images. Cameras may be infrared (IR) cameras (which capture IR data) or RGB cameras (which capture visible spectrum data). Commonly, an IR camera is used in conjunction with light-emitting diodes (LEDs) for eye illumination, due to the high level of accuracy that can be achieved for gaze tracking. In such IR-based systems, usually two or more IR LEDs are used to illuminate the eye. Use of two or more IR LEDs for gaze estimation builds redundancy into the system, ensuring that at least two corneal reflections are generated in a captured image over a range of eye positions. However, there is benefit in using the fewest number of IR LEDs possible for eye-tracking, as hardware circuitry complexity and image noise can increase as the number of LEDs increases, negatively impacting the performance of the gaze estimation system.

Therefore, it would be useful to provide a solution that enables 3D gaze estimation using a single corneal reflection.

US 2019/213402 A1 discloses a computer-implemented method for estimating head pose angles of a user including determining a first rotation between a first head pose axis associated with a first image of a plurality of images of the user and a camera axis associated with a camera taking the images. A second rotation is determined between a second head pose axis associated with a second image of the user and the camera axis. The first and second head pose axes are determined based on light reflections within the plurality of images. A head pose angle of the user can be estimated based on the first rotation and the second rotation. An alert can be generated based on the estimated head pose angle.

SUMMARY

The invention is defined by an eye-tracking system of claim 1. Preferred embodiments are defined in the dependent claims. In various examples, the present disclosure describes methods and systems for estimating an individual's gaze direction using a position of a single corneal reflection, a position of a pupil center and positional information for an individual's head as inputs. Specifically, an image of a user including at least an image of a portion of the user's head is obtained from an infrared (IR) camera and a 2D position of a corneal reflection is estimated in the image. Positional information for the user's head is determined from the IR camera image. A 3D position of a cornea center for the user's eye is then estimated based on the 2D position of the corneal reflection in the image, a position of an IR light source (e.g. IR LED) and the positional information of the user's head. A 2D position of the pupil center for the user's eye is estimated in the IR camera image and a 3D position of a pupil center is then estimated based on the 2D position of the pupil center and the 3D position of the cornea center. Finally, a 3D gaze vector representing a gaze direction is estimated based on the 3D position of the cornea center and the 3D position of the pupil center. The disclosed system may help to overcome challenges associated with gaze estimation performance for systems requiring two or more corneal reflections, for example, under conditions of extreme head movement.

The disclosed systems and methods enable 3D gaze estimation using a single corneal reflection. This enables improved gaze estimation performance under conditions where detection of two or more corneal reflections may be difficult, for example, under conditions of extreme movement or during extreme head positions, among other possibilities.

In various examples, the present disclosure provides the technical effect that a gaze direction, in the form of a 3D gaze vector is estimated. Inputs obtained from an IR camera in the form of face or eye images along with positional information of the individual's head are inputted into a gaze estimation system to estimate the point of gaze either on a screen or in a surrounding environment.

In some examples, the present disclosure provides the technical advantage that a gaze direction is estimated using only one corneal reflection, rather than two or more corneal reflections.

Examples of the present disclosure may enable improved gaze estimation performance in conditions of extreme head movement, for example, where it may be difficult to accurately capture two or more corneal reflections in an image. Accordingly, requiring only one corneal reflection in an input image may reduce noise and data loss, for example, reduce the frequency of instances where an image cannot be used to compute a gaze direction. In examples, more than one IR LED may be included in an eye-tracking system however images where only one corneal reflection is visible (e.g. at extreme head positions) may be used to compute a gaze direction, where they would have been unusable for systems requiring two or more corneal reflections.

In some examples, the present disclosure provides the technical advantage that tracking only one corneal reflection instead of multiple reflections means that hardware configuration required for estimating a gaze direction are simpler, for example, hardware circuitry complexity associated with synchronizing IR LEDs with the camera shutter is reduced. System configuration may also benefit from greater flexibility, for example, an IR LED may be placed in a wider range of locations. Similarly, the required computational resources for estimating a gaze direction are less complex.

In an example aspect, the present disclosure describes a method. The method includes: obtaining an image of a user, the image including an image of an eye of the user; estimating a position of a corneal reflection based on the image; determining a positional information for the user's head based on the image; and estimating a position of a cornea center for the user's eye based on the position of the corneal reflection and the positional information.

Optionally, the image is obtained by using an IR camera and at least one IR LED, and the image is IR camera image.

In the preceding example aspect of the method, the method further comprising: estimating a position of a 3D pupil center for the user's eye based on the IR camera image and the position of the cornea center

In the preceding example aspect of the method, the method further comprising: estimating a gaze vector representing the user's gaze direction based on the position of the cornea center and the position of the 3D pupil center.

In the preceding example aspect of the method, wherein estimating the gaze vector representing the user's gaze direction comprises: estimating an optical axis of the user's eye based on the position of the cornea center and the position of the 3D pupil center; and estimating the gaze vector based on the optical axis and the plurality of calibration parameters.

In any of the preceding example aspects of the method, wherein the positional information for the user's head includes a head pose of the user.

In the preceding example aspect of the method, wherein obtaining the head pose of the user comprises: estimating one or more face landmarks corresponding to a face of the user, based on the IR camera image; fitting the estimated one or more face landmarks to a 3D face model; and estimating a head pose based on the 3D face model.

In any of the preceding example aspects of the method, wherein the IR camera is positioned at a distance from the user and the IR camera image is a face image.

In some example aspects of the method, wherein the IR camera image is an eye image and the positional information for a user's head is a distance of a user's eye from the IR camera, the distance of the user's eye from the IR camera being obtained from a head mounted device.

In an example aspect of the method, wherein the IR camera image of a user includes a bright pupil.

In some example aspects of the method, wherein the estimated gaze vector is a 3D gaze vector.

In some aspects, the present disclosure describes an eye-tracking system. The system comprises: one or more processors; and a memory storing machine-executable instructions which, when executed by the processor device, cause the system to: obtain an image of a user, the image including an image of an eye of the user; estimate a position of a corneal reflection based on the image; determine a positional information for the user's head based on the image; and estimate a position of a cornea center for the user's eye based on the position of the corneal reflection and the positional information.

Optionally, the system further comprises an IR camera; a first IR LED; and the image is obtained by the IR camera and the first IR LED; the image is IR camera image.

In some example aspects, the present disclosure describes a non-transitory computer readable medium storing instructions thereon. The instructions, when executed by a processor, cause the processor to: perform any of the preceding example aspects of the method.

BRIEF DESCRIPTION OF THE DRAWINGS

Reference will now be made, by way of example, to the accompanying drawings which show example embodiments of the present application, and in which:

FIG. 1 is a schematic block diagram of an eye-tracking system suitable for implementation of examples described herein;

FIG. 2 is a block diagram illustrating an example hardware structure of a computing system that may be used for implementing methods to estimate a gaze vector representing a gaze direction, in accordance with examples of the present disclosure;

FIG. 3 is a block diagram illustrating an example architecture of a gaze estimation system, in accordance with example embodiments of the present disclosure;

FIG. 4 is a block diagram illustrating an example architecture of a head pose estimator, in accordance with example embodiments of the present disclosure;

FIG. 5 is a block diagram illustrating an example architecture of a gaze estimator, in accordance with example embodiments of the present disclosure;

FIG. 6A illustrates an example embodiment of the eye-tracking system, in accordance with examples of the present disclosure;

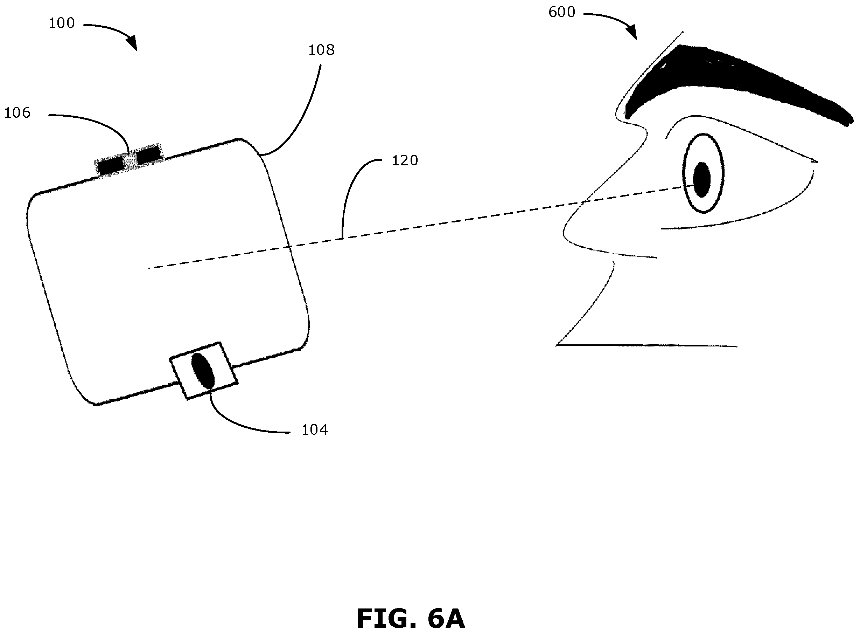

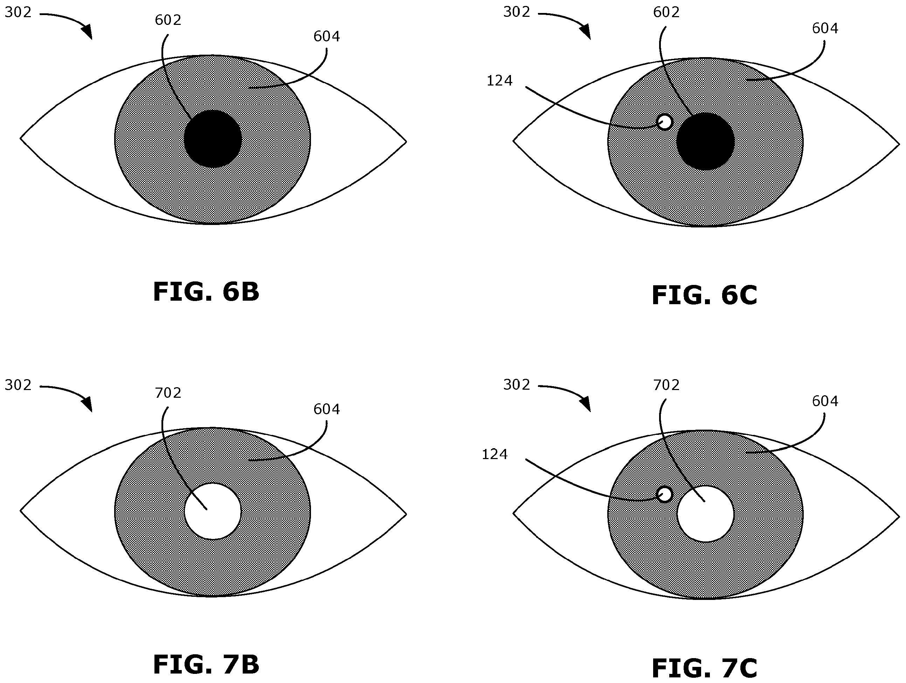

FIG.s 6B and 6C illustrate example IR camera images obtained for the embodiment of FIG 6A, in accordance with examples of the present disclosure;

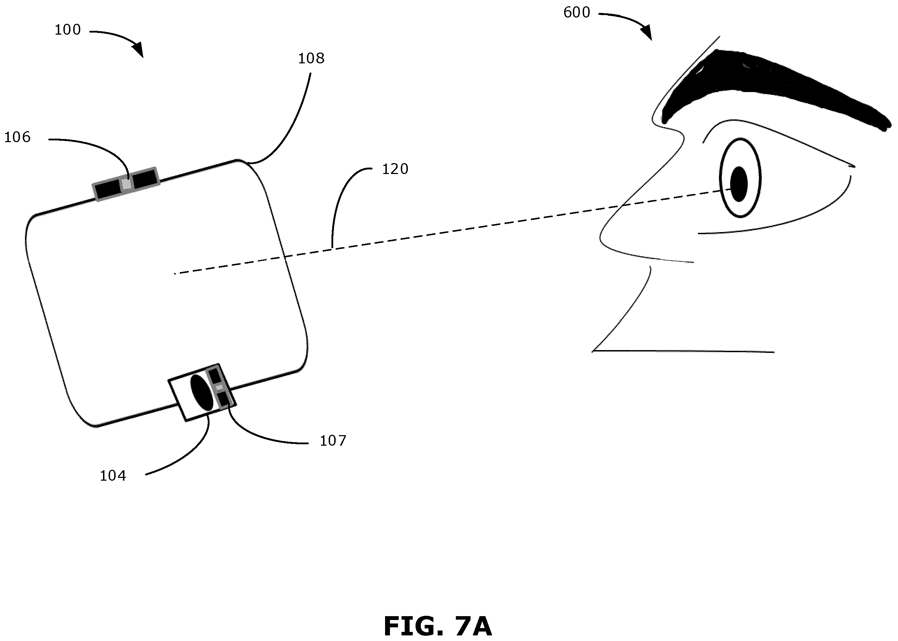

FIG. 7A illustrates an example embodiment of the eye-tracking system, in accordance with examples of the present disclosure;

FIG.s 7B and 7C illustrate example IR camera images obtained for the embodiment of FIG 7A, in accordance with examples of the present disclosure;

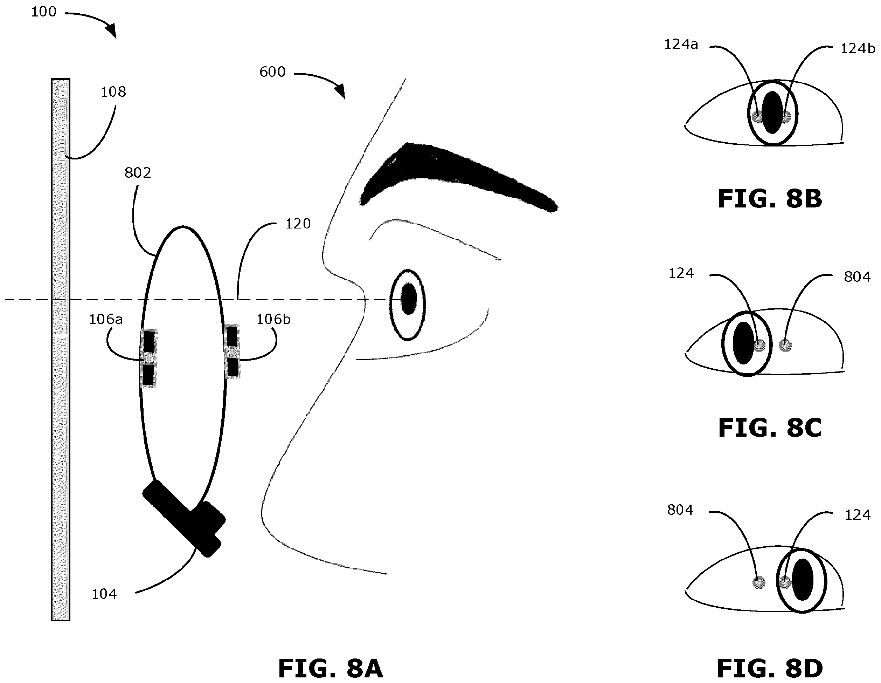

FIG. 8A illustrates an example embodiment of the eye-tracking system, in accordance with examples of the present disclosure;

FIG.s 8B, 8C and 8D illustrate example IR camera images obtained for the embodiment of FIG 8A, in accordance with examples of the present disclosure;

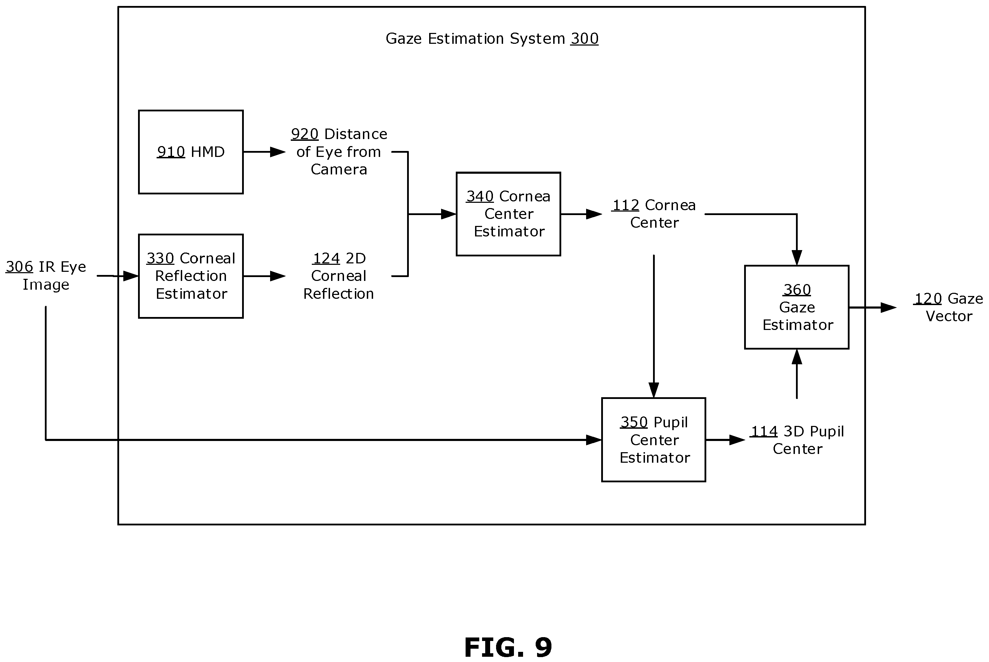

FIG. 9 is a block diagram illustrating an example architecture of the gaze estimation system for use in conjunction with the embodiment of FIG 8A, in accordance with example embodiments of the present disclosure; and

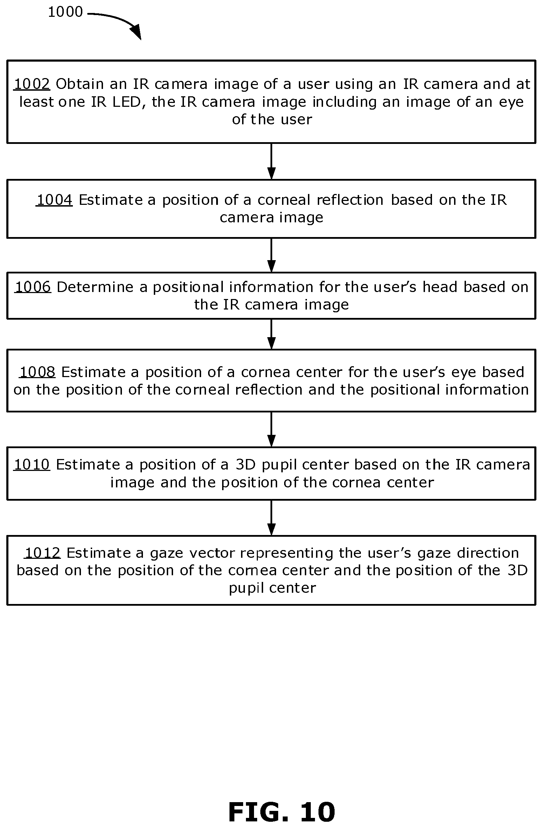

FIG. 10 is a flowchart illustrating steps of an example method for estimating a gaze vector representative of a user's gaze direction, in accordance with examples of the present disclosure.

## DETAILED DESCRIPTION

The following describes example technical solutions of this disclosure with reference to accompanying drawings.

In various examples, the present disclosure describes methods and systems for estimating an individual's gaze direction using a position of a single corneal reflection, a position of a pupil center and positional information for an individual's head as inputs. Specifically, an image of a user including at least an image of a portion of the user's head is obtained from an infrared (IR) camera and a 2D position of a corneal reflection is estimated in the image. Positional information for the user's head is determined from the IR camera image. A 3D position of a cornea center for the user's eye is then estimated based on the 2D position of the corneal reflection in the image, a position of an IR light source (e.g. IR LED) and the positional information of the user's head. A 2D position of the pupil center for the user's eye is estimated in the IR camera image and a 3D position of a pupil center is then estimated based on the 2D position of the pupil center and the 3D position of the cornea center. Finally, a 3D gaze vector representing a gaze direction is estimated based on the 3D position of the cornea center and the 3D position of the pupil center. The disclosed system may help to overcome challenges associated with gaze estimation performance for systems requiring two or more corneal reflections, for example, under conditions of extreme head movement.

To assist in understanding the present disclosure, some existing techniques for gaze tracking are now discussed.

Existing camera-based eye-tracking systems are commonly used to estimate a user's gaze direction by capturing an image and providing the image to a gaze estimation algorithm to estimate the gaze direction. Gaze estimate may be determined as a 2D gaze point (e.g. on a display screen) or a 3D gaze vector. A 2D gaze point may be determined by estimated by intersecting a 3D gaze vector with the plane of the display screen. 3D gaze estimation systems provide additional benefits over 2D eye-tracking systems as they can be used to track a gaze direction in environments without a display screen, for example, in a vehicle.

Typical hardware configuration for eye-tracking systems include remote systems and head mounted systems. In remote eye-tracking systems, hardware components including the camera and any LEDs are placed far away from the user, whereas in head mounted eye-tracking systems, hardware components are placed inside a head mounted device such as an Augmented Reality (AR) or Virtual Reality (VR) device causing the camera and any LEDs to be positioned in close proximity with the eye. Cameras used in eye-tracking systems may include infrared (IR) cameras (which capture IR data) or RGB cameras (which capture visible spectrum data). IR based cameras and LEDs are often preferred over visible light since IR light doesn't interfere with human vision, and IR-based systems can operate in most environments, including night time. For these and other reasons, IR-based eye-tracking systems operate with high accuracy.

A primary purpose of IR LEDs in an eye-tracking system is for providing even illumination across the captured image which results in increased signal to noise ratio (SNR) in the captured image. High SNR means that the captured image is of good quality, improving the performance of the gaze algorithms. Another important use of IR LEDs in eye-tracking systems is to create corneal reflections. Corneal reflections are virtual images of the reflections of the IR LEDs on the cornea of the eye, that are formed behind the corneal surface and captured as a glint in an IR image. Locations of corneal reflections in the captured image are important eye features used by stateof-the-art gaze algorithms. The number of corneal reflections generated for a user's eye is dependent on the number of IR LEDs, and various gaze algorithms require varying numbers of corneal reflections in an image to determine a gaze direction.

Gaze estimation algorithms can be categorized based on the type of algorithm used to estimate the point of gaze or the gaze direction, as well as number of corneal reflections used in the processing: 1) appearance-based, 2) feature-based 3) geometrical eye model-based and 4) cross ratio-based. Appearance-based methods use the appearance of the face or eye in the image to learn a direct mapping between the input image and the gaze direction or point of gaze Appearance-based methods compute gaze position by leveraging machine learning (ML) techniques on images captured by the eye-tracking system. Appearance-based methods do not require any information regarding corneal reflections. As described in Wu, Zhengyang, et al. "Magiceyes: A large scale eye gaze estimation dataset for mixed reality," arXiv preprint arXiv: 2003.08806 (2020), the best appearance-based methods may perform with an accuracy of 2-3 degrees. Improved accuracy with appearance-based methods can be achieved by retraining the ML network for every subject, however this may not be practical. Appearance-based techniques also require large training datasets.

Feature-based methods use the spatial location of features extracted from images of the face (e.g. the vector between one corneal reflection and the pupil center) to estimate gaze direction. An example of feature-based methods is described in Zhu, Zhiwei, and Qiang Ji, "Novel eye gaze tracking techniques under natural head movement," IEEE Transactions on biomedical engineering 54,12 (2007): 2246-2260. Estimating eye features from a face image is a challenging process, particularly at extreme head angles. Feature-based methods can also only estimate 2D gaze and require a display screen to be present in the system. Cross ratio-based methods, an example of which is described in Fan, Shuo, Jiannan Chi, and Jiahui Liu, "A Novel Cross-Ratio Based Gaze Estimation Method," 2022 International Conference on Machine Learning and Knowledge Engineering (MLKE), IEEE, 2022, make use of 4 or more corneal reflections along with pupil center to estimate gaze direction, however they are sensitive to head movements.

Geometrical model-based methods use a 3D model of the eye to estimate a gaze direction (e.g. visual axis), where the visual axis of the eye is determined to be the vector that connects the nodal point of the eye and the fovea and the point of gaze is the intersection of the eye's visual axis and the scene of interest. Commonly, geometrical eye-model methods use the coordinates of the pupil and two corneal reflections to compute a 3D gaze direction. An example of a geometrical model-based method is described in Guestrin, E. D., & Eizenman, M. (2006), General theory of remote gaze estimation using the pupil center and corneal reflections. IEEE Transactions on biomedical engineering, 53(6), 1124-1133. Geometrical eye model-based methods are insensitive to head movement and can achieve better accuracy than previously described categories of gaze methods, for example, achieving accuracy better than 1 degree in desktop, smartphone and head mounted based systems. Such methods usually use more than two IR LEDs to ensure that at least 2 corneal reflections are present in the captured image over a range of all possible eye positions. However, there is a benefit to minimize the number of IR LEDs, for example, in reducing the hardware complexity of the eye-tracking system and reducing computational resources required for implementing the gaze tracking algorithm.

The present disclosure describes examples that may help to address some or all of the above drawbacks of existing technologies.

In the present disclosure, references to different coordinate systems may identify specific local or global coordinate systems, for example, coordinates expressed in an image coordinate system, a camera coordinate system and an eye coordinate system may all describe positions relative to a local coordinate system which may be converted to coordinates describing a position relative to the world coordinate system.

In the present disclosure, a "gaze vector" or "visual axis" can mean: the vector representing the gaze direction of an individual. In a geometrical eye model-based method, the visual axis of the eye is the vector that connects the nodal point of the eye and the fovea. In examples, a 3D gaze vector may be expressed in the eye coordinate system.

In the present disclosure, a "Point-of-gaze (POG)" can mean: the object or location within a scene of interest where an individual is looking, or more specifically, the intersection of the eye's visual axis with a scene of interest. In other examples, a POG may correspond to a location on a display screen where a visual axis intersects the 2D display screen.

In the present disclosure, an "optical axis" the line connecting pupil center and corneal center in a 3D geometric eye model. In examples, optical axis may be expressed in world coordinate system.

In the present disclosure, a "corneal reflection (CR)" is a virtual image of a reflection of an IR LED on the cornea, where the CR is formed behind the corneal surface, and captured in the IR camera image. In examples, a CR may be a set of 2D coordinates describing a position of the CR within the IR camera image, and expressed in the image coordinate system. In examples, the CR may also be referred to as a "glint".

In the present disclosure, a "cornea center" is a 3D position of the center point of the cornea. In examples, the cornea center may be a set of 3D coordinates describing a position of the center of the cornea within the world coordinate system. In examples, the cornea center may also act as the origin of the eye coordinate system.

In the present disclosure, a "3D pupil center" is a 3D position of the center point of the pupil. In examples, the 3D pupil center may be a set of 3D coordinates describing a position of the center of the pupil within the world coordinate system.

In the present disclosure, a "2D pupil center" is a 2D position of the center point of the pupil within an IR camera image. In examples, the 2D pupil center may be a set of 2D coordinates describing the center of the pupil within the image coordinate system.

In the present disclosure, a "remote eye-tracking system" can mean: an eye-tracking system in which the hardware components including the camera and any LEDs are placed far away from the user.

In the present disclosure, a head mounted eye-tracking system" can mean: an eye-tracking system in which the hardware components including the camera and any LEDs are placed inside a head mounted device such as an Augmented Reality (AR) or Virtual Reality (VR) device, causing the camera and any LEDs to be positioned in close proximity with the eye.

Other terms used in the present disclosure may be introduced and defined in the following description.

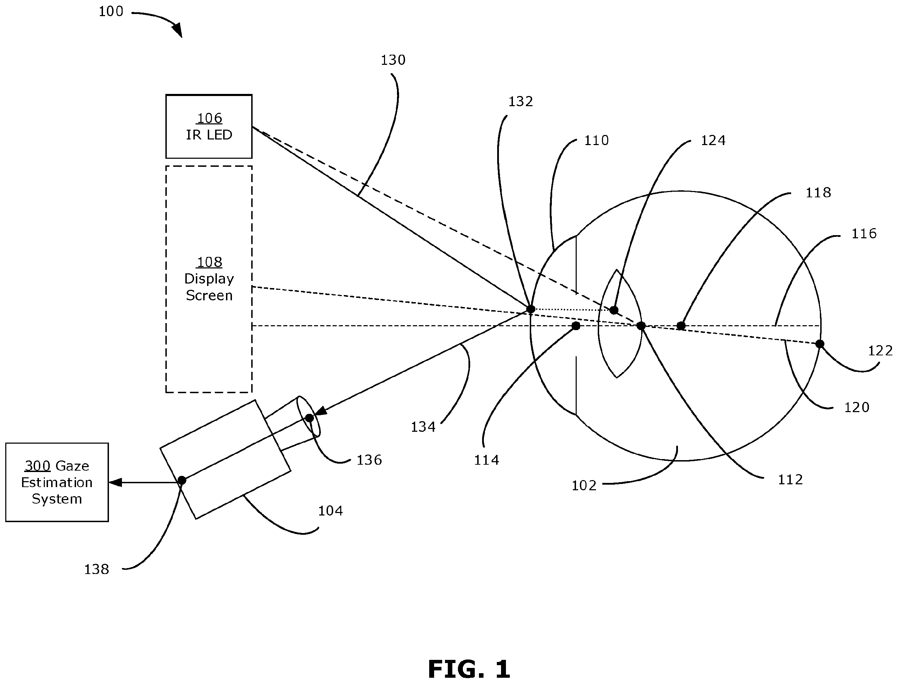

FIG. 1 shows an eye-tracking system 100 including eye-tracking equipment and a 3D geometrical eye-model of a user's eye, suitable for implementation of examples described herein. An example of a 3D geometrical eye-model that can be implemented in example embodiments is described in: Guestrin, E. D., & Eizenman, M. (2006). General theory of remote gaze estimation using the pupil center and corneal reflections. IEEE Transactions on biomedical engineering, 53(6), 1124-1133. The eye-tracking system 100 is an illustrative example of a system to which the systems, methods, and processor-readable media described herein can be applied, in accordance with examples of the present disclosure.

In some embodiments, for example, the eye-tracking system 100 includes an image capturing device, for example, an infrared (IR) camera 104 for capturing an IR camera image 302 of a user, and an illumination device, for example, an IR LED 106, for illuminating at least a portion of the head of a user, including an eye 102 of a user. In examples, the IR camera image 302 may include an image of at least a portion of the user's head, including an eye 102 of the user. In examples, the IR camera 104 may communicate with a gaze estimation system 300 to enable estimating a gaze vector 120 representing a gaze direction of the user. Optionally, a display screen 108 may include a display of an electronic device, such as a desktop or laptop computer, a mobile communications device, or a virtual reality/augmented reality (VR/AR) device, among others.

In some embodiments, for example, the user's eye 102 may be represented as a 3D geometric model of an eye, including a cornea surface 110, a position of the cornea center $c$ 112, and a position of a pupil center $p$ 114. An optical axis 116 of the eye 102 may be an axis passing through the center of rotation of the eye 118, the cornea center 112 and the pupil center 114. In examples, a visual axis (e.g. the gaze vector 120) representing a gaze direction may be an axis connecting the cornea center 112 with the center of the fovea 122. In the present disclosure, the term visual axis may be interchangeable with the term gaze vector.

In some embodiments, for example, the IR LED 106 may illuminate the eye 102 and generate a corneal reflection 124 (e.g. a glint). In modeling the IR LED 106 as a point light source $i$ and modeling the IR camera 104 as a pinhole camera $j$, an incident ray 130 coming from the IR LED $l_i$ 106 may reflect at a point of reflection $q_i$ 132 along the cornea surface 110, and a reflected ray 134 may pass through the nodal point o 136 of the IR camera 104 to intersect the image plane of the IR camera 104 at a point $u_{ij}$ 138. In examples, a corneal reflection 124 may be a virtual image of the reflection of the IR LED 106 on the cornea surface, where point $u_{ij}$ 138 represents the location of the corneal reflection in the captured IR camera image 302.

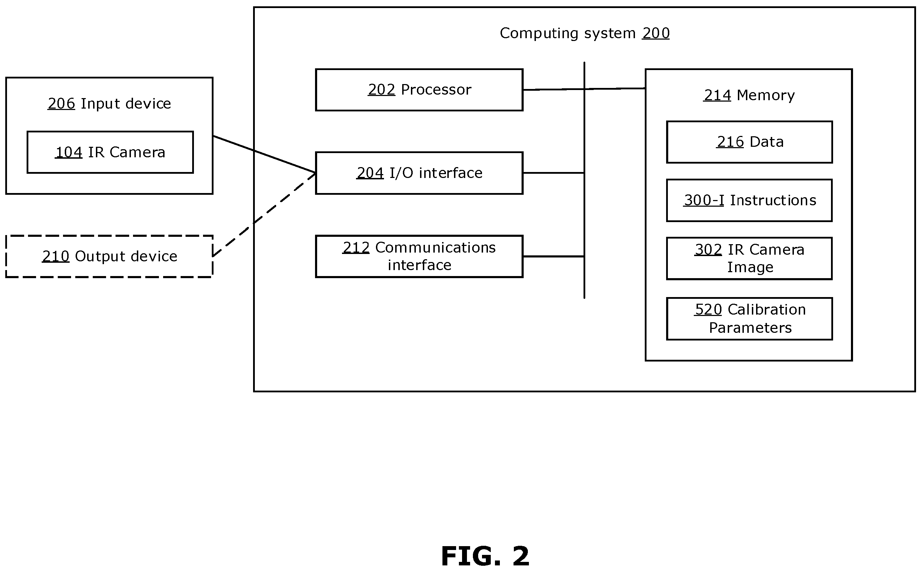

FIG. 2 is a block diagram illustrating an example hardware structure of a computing system 200 that is suitable for implementing embodiments described herein. Examples of the present disclosure may be implemented in other computing systems, which may include components different from those discussed below. The computing system 200 may be used to execute instructions for estimating a gaze vector 120 representing a gaze direction, using any of the examples described herein. The computing system 200 may also be used to train blocks of the gaze estimation system 300, or blocks of the gaze estimation system 300 may be trained by another computing system.

Although FIG. 2 shows a single instance of each component, there may be multiple instances of each component in the computing system 200. Further, although the computing system 200 is illustrated as a single block, the computing system 200 may be a single physical machine or device (e.g., implemented as a single computing device, such as a single workstation, single end user device, single server, etc.), and may include mobile communications devices (smartphones), laptop computers, tablets, desktop computers, vehicle driver assistance systems, smart appliances, wearable devices, assistive technology devices, medical diagnostic devices, virtual reality devices, augmented reality devices, Internet of Things (IoT) devices, interactive kiosks, advertising and interactive signage, and educational tools, among others.

The computing system 200 includes at least one processor 202, such as a central processing unit, a microprocessor, a digital signal processor, an application-specific integrated circuit (ASIC), a field-programmable gate array (FPGA), a dedicated logic circuitry, a dedicated artificial intelligence processor unit, a graphics processing unit (GPU), a tensor processing unit (TPU), a neural processing unit (NPU), a hardware accelerator, or combinations thereof.

The computing system 200 may include an input/output (I/O) interface 204, which may enable interfacing with an input device 206 and/or an optional output device 210. In the example shown, the input device 206 (e.g., a keyboard, a mouse, a microphone, a touchscreen, and/or a keypad) may also include an IR camera 104. In the example shown, the optional output device 210 (e.g., a display, a speaker and/or a printer) are shown as optional and external to the computing system 200. In other example embodiments, there may not be any input device 206 and output device 210, in which case the I/O interface 204 may not be needed.

The computing system 200 may include an optional communications interface 212 for wired or wireless communication with other computing systems (e.g., other computing systems in a network). The communications interface 212 may include wired links (e.g., Ethernet cable) and/or wireless links (e.g., one or more antennas) for intra-network and/or inter-network communications.

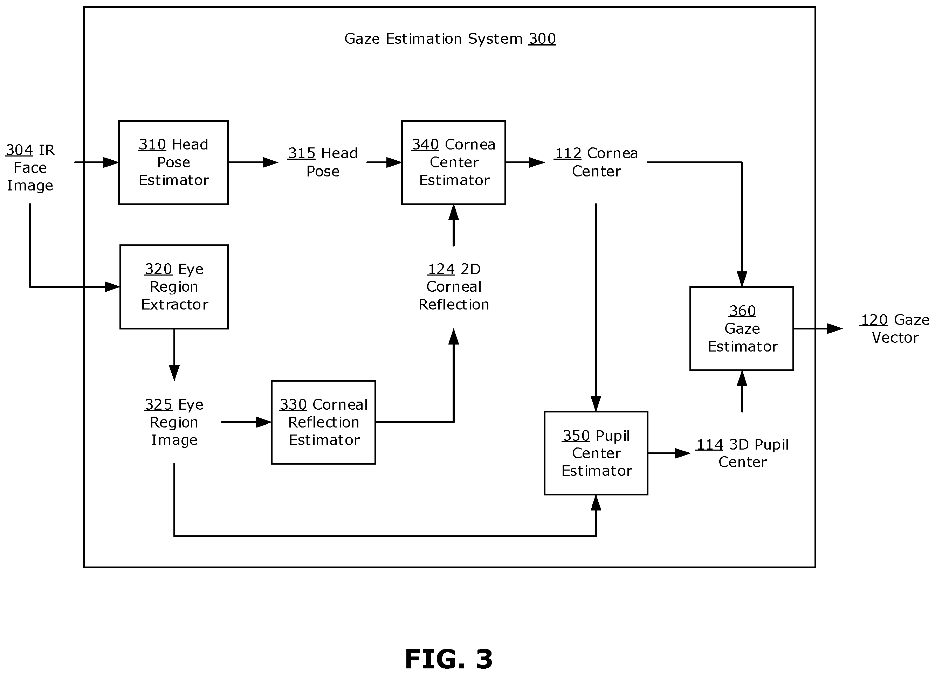

The computing system 200 may include one or more memories 214 (collectively referred to as "memory 214"), which may include a volatile or non-volatile memory (e.g., a flash memory, a random access memory (RAM), and/or a read-only memory (ROM)). The non-transitory memory 214 may store instructions for execution by the processor 202, such as to carry out examples described in the present disclosure. For example, the memory 214 may store instructions for implementing any of the networks and methods disclosed herein. The memory 214 may include other software instructions, such as for implementing an operating system (OS) and other applications/functions. The instructions can include instructions 300-I for implementing and operating the gaze estimation system 300 described below with reference to FIGs 3, 4 and 5.

The memory 214 may also store other data 216, information, rules, policies, and machine-executable instructions described herein, including an IR camera image 302 captured by the IR camera 104 or eye-tracking system calibration parameters 520 for a user.

In some examples, the computing system 200 may also include one or more electronic storage units (not shown), such as a solid state drive, a hard disk drive, a magnetic disk drive and/or an optical disk drive. In some examples, data and/or instructions may be provided by an external memory (e.g., an external drive in wired or wireless communication with the computing system 200) or may be provided by a transitory or non-transitory computer-readable medium. Examples of non-transitory computer readable media include a RAM, a ROM, an erasable programmable ROM (EPROM), an electrically erasable programmable ROM (EEPROM), a flash memory, a CD-ROM, or other portable memory storage. The storage units and/or external memory may be used in conjunction with memory 214 to implement data storage, retrieval, and caching functions of the computing system 200. The components of the computing system 200 may communicate with each other via a bus, for example.

FIG. 3 is a block diagram illustrating an example architecture of the gaze estimation system 300 that may be used to implement methods to estimate a gaze vector 120 representing a gaze direction, in accordance with example embodiments of the present disclosure.

In some examples, the gaze estimation system 300 receives an IR camera image 302 (e.g. an IR face image 304) and outputs an estimated gaze vector 120 including gaze angles, where the gaze vector 120 represents a gaze direction of a user. The IR camera image 302 may be captured by the IR camera 104 on the computing system 200 or the IR camera image 302 may be a digital image taken by another IR camera on another electronic device and communicated to the computing system 200 (e.g., in the case where the computing system 200 provides a gaze estimation service to other devices). In examples, the IR camera image 302 may be extracted from video images captured by the IR camera 104. In some embodiments, the IR camera image 302 may be obtained from a remote IR camera 104 positioned at a distance from a user 600 or the IR camera image 302 may be obtained in close proximity to a user by an IR camera 104 within a head mounted device (HMD) 910. In some embodiments, for example, the IR camera image 302 may be an IR face image 304, where the IR face image 304 is an IR image encompassing an user's face including features such as the eyes, nose, mouth, and chin, among others. In other embodiments, the IR camera image 302 may be an IR eye image 306, where the IR eye image 306 is an IR image encompassing a user's eye.

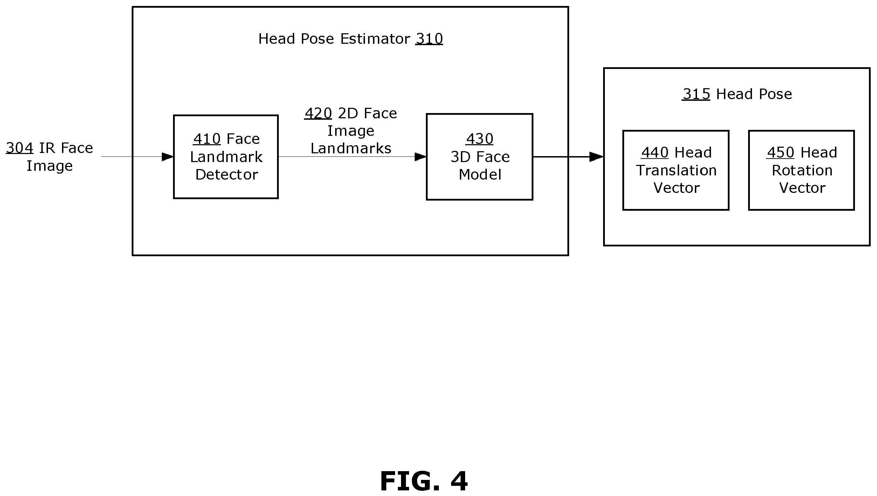

In some embodiments, for example, a head pose estimator 310 of the gaze estimation system 300 may receive the IR camera image 302 as an IR face image 304 and may output positional information for the user's head as a head pose 315, as described in the discussion of FIG. 4 below.

FIG. 4 is a block diagram illustrating an example architecture of a head pose estimator 310, in accordance with example embodiments of the present disclosure. In examples, a face landmark detector 410 of the head pose estimator 310 receives the IR face image 304 and outputs 2D face image landmarks 420. In examples, the face landmark detector 410 may be any suitable pre-trained machine-learning based face landmark detection algorithm, for example Google MediaPipe, or others. The face landmark detector 410 may identify 2D landmarks in the IR face image 304 and may apply contours around landmarks such as the eyes, nose, mouth, and chin, among others. In some embodiments, for example, the 2D face image landmarks 420 may be used with a 3D face model 430, for example, by fitting the 3D face model 430 to the 2D face image landmarks 420, in order to estimate a head pose 315 of the user's head in 3D space. In examples, the head pose 315 may include both a head translation vector $(t_x, t_y, t)$ 440 and a head rotation vector $(\theta_x, \theta_y, \theta_z)$ 450. In examples, a distance $k_q$ representing a distance between a user's eye and the IR camera 104, may be determined in the world coordinate system using the translation vector 440 as shown in equation 1 below.

$$k_q = \sqrt[2]{t_x^2 + t_y^2 + t_z^2} \ \#(1)$$

Returning to FIG. 3, in some embodiments, an eye region extractor 320 of the gaze estimation system 300 may also receive the IR camera image 302 as an IR face image 304 and may extract a portion of the IR face image 304 corresponding to an eye region of a user as an eye region image 325. In examples, the eye region extractor 320 may use any suitable pre-trained machine-learning based face landmark detection algorithm, for example Google MediaPipe, or others, to identify one or both eyes in the IR face image 304. In examples, the eye region extractor 320 may apply contours around the user's eyes in the IR face image 304, and an eye region image 325 including an image of one of the user's eyes may be cropped from the IR face image 304.

In examples, the eye region image 325 may be input to a corneal reflection estimator 330 to obtain at least one position of a corneal reflection 124 within the eye region image 325, where a corneal reflection 124 is a virtual image of the reflection of a respective IR LED 106 on the cornea surface of the user's eye, and where the number of corneal reflections 124 depends on the number of IR LEDs 106. An example of a corneal reflection estimator 330 that can be implemented in example embodiments is described in: Chugh, S. et al. "Detection and Correspondence Matching of Corneal Reflections for Eye Tracking Using Deep Learning" in Proceedings of the 25th International Conference on Pattern Recognition (ICPR), IEEE, 2020.

In some embodiments, the position of at least one corneal reflection 124 may be input to a cornea center estimator 340. In examples, the cornea center estimator 340 may also receive positional information as the head pose 315, as well as a location of the light source (IR LED 106). In examples, the cornea center estimator 340 may determine a position of a cornea center c 112, for example, as 3D coordinates $(x,y,z)$ based on the position of the at least one corneal reflection 124, the light source location and the positional information. In examples, the cornea center estimator 340 may be a block that computes the position of the cornea center c 112 based on equations 2-5 below.

$$q_i = o + k_{qi}\frac{o - u_i}{\|o - u_i\|}, for\ some\ k_{qi}, \#(2)$$

where $k_{qi}$ represents the distance between the point of reflection $q_i$ 132 and the nodal point $o$ 136 of the IR camera 104, as computed in equation 1 above, and $u_i$ represents the position of the corneal reflection 124 in the image obtained by IR camera 104. For any point falling on the cornea, the following condition may be satisfied:

$$R = \|q_i - c\| \ \#(3)$$

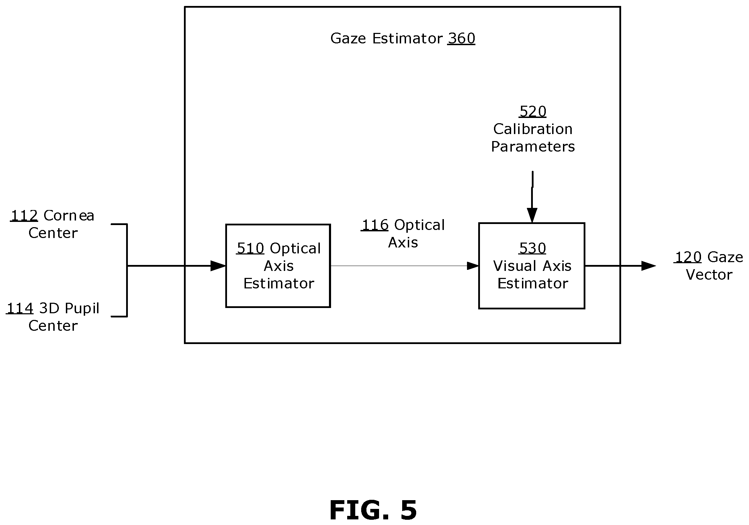

where $R$ is the radius of the cornea. In examples $R$ may be estimated as 7.8 mm, as described in: Guestrin, E. D., & Eizenman, M. (2006). General theory of remote gaze estimation using the pupil center and corneal reflections. IEEE Transactions on biomedical engineering, 53(6), 1124-1133. In other examples, $R$ may be determined during a calibration procedure, for example, as described with respect to FIG. 5. In examples, the incident ray 130 from the IR LED $l_i$ 106, the reflected ray 134 from the cornea surface and the normal at the point of reflection $q_i$ 132 are in the same plane, therefore equation 4 is presented as:

$$(l_i - o) \times (q_i - o) \cdot (c - o) = 0 \ \#(4)$$

where $l_i$ is the location of the center of the light source (e.g. IR LED 106) and $o$ is the nodal point 136 of the IR camera 104. Finally, at the point of reflection $q_i$ 132, the angle between the incident ray and the normal to the cornea surface is equal to the angle between the reflected ray 134 and the normal to the cornea surface, therefore equation 5 is presented as:

$$\left(\frac{l_i - q_i}{\|l_i - q_i\|} - \frac{o - q_i}{\|o - q_i\|}\right) \cdot (q_i - c) = 0 \ \#(5)$$

In some embodiments, for example, the cornea center estimator 340 may compute the 3D coordinates of the cornea center $c$ 112 by solving for the cornea center $c$ 112 using equations 2-5 and given the value of $k_{qi}$ as calculated in equation 1 and the radius of the cornea R.

In some embodiments, for example, the eye region image 325 and the position of the cornea center 112 may be input to a pupil center estimator 350 to generate a position of a 3D pupil center 114. An example of a pupil center estimator 350 that can be implemented in example embodiments is described in: Guestrin, E. D., & Eizenman, M. (2006). General theory of remote gaze estimation using the pupil center and corneal reflections. IEEE Transactions on biomedical engineering, 53(6), 1124-1133.

In some embodiments, for example, the cornea center 112 and the 3D pupil center 114 may be input to a gaze estimator 360 to generate a gaze vector 120, as described in the discussion of FIG. 5 below.

FIG. 5 is a block diagram illustrating an example architecture of a gaze estimator 360, in accordance with example embodiments of the present disclosure. In examples, the gaze estimator 360 receives the position of the cornea center 112 and the position of the 3D pupil center 114 and outputs a gaze vector 120 representing a gaze direction. In some embodiments, for example, the gaze estimator may include an optical axis estimator 510 block for computing an optical axis 116 and a visual axis estimator 530 block for computing the gaze vector 120 based on the optical axis and a plurality of subject-specific calibration parameters 520. An example of a gaze estimator 360 that can be implemented in example embodiments is described in: Guestrin, E. D., & Eizenman, M. (2006). General theory of remote gaze estimation using the pupil center and corneal reflections. IEEE Transactions on biomedical engineering, 53(6), 1124-1133.

In examples, calibration parameters 520 may be obtained and stored for a user during a one-time calibration procedure, for example, where a user looks at one or more target points with known locations on a display screen 108 while a plurality of IR camera images 302 are captured for each respective target point. In some embodiments, for example, the gaze estimation system 300 may predict a plurality of gaze vectors 120 from the plurality of IR camera images 302 obtained during the calibration procedure, and may compare the predicted gaze vectors 120 to each of the one or more target points with known locations. In examples, an optimization algorithm may be used to obtain the plurality of user-specific calibration parameters 520 that minimizes the error between the predicted gaze vectors 120 and the target points. In examples, the optimization algorithm may include a least squares method or another optimization method may be used. In some examples, the plurality of calibration parameters 520 may include an angular offset between the optical axis and the visual axis of the user's eye, and/or the calibration parameters 520 may include parameters representing the radius of the user's eye $R$, the distance between the 3D pupil center 114 and the cornea center 112, or the effective index of refraction of the aqueous humor and cornea of the user's eye, among other subject-specific parameters. In examples, the gaze vector 120 may be computed from the optical axis 116 using the angular offset between the optical axis and the visual axis of the user's eye.

FIG. 6A illustrates an example embodiment of the eye-tracking system 100, where the eye-tracking system 100 is a remote eye-tracking system, and includes one IR LED 106 located at a distance from the IR camera 104, in accordance with examples of the present disclosure. In the present embodiment, a user 600 interacts with the remote eye-tracking system 100 and a gaze vector 120 represents the direction of the user's gaze. An optional display screen 108 is shown to demonstrate screen-based interaction. In the present embodiment, the IR LED 106 is positioned far from the IR camera 104 for generating a corneal reflection 124. FIG.s 6B and 6C illustrate example IR camera images 302 obtained for the embodiment of FIG 6A, for example, corresponding to a user's eye, in accordance with examples of the present disclosure. In examples, the IR camera images 302 may include features of the eye such as a pupil (e.g. a dark pupil 602) and an iris 604. As shown in FIG. 6C, one or more corneal reflections 124 may also be visible in the IR camera image 302.

FIG. 7 illustrates an example embodiment of the eye-tracking system 100, where the eye-tracking system 100 is a remote eye-tracking system, and includes a first IR LED 106 located at a distance from the IR camera 104 and a second IR LED 107 located in proximity to the IR camera 104, in accordance with examples of the present disclosure. In the present embodiment, a user 600 interacts with the remote eye-tracking system 100 and gaze vector 120 represents the direction of the user's gaze. An optional display screen 108 is shown to demonstrate screen-based interaction. A In the present embodiment, the first IR LED 106 is positioned far from the IR camera 104 for generating a corneal reflection 124 and the second IR LED 107 is located in proximity to the IR camera 104 for creating a bright pupil effect in the IR camera image 302. FIG.s 7B and 7C illustrate example IR camera images 302 obtained for the embodiment of FIG 7A, for example, corresponding to a user's eye, in accordance with examples of the present disclosure. In examples, the IR camera images 302 may include features of the eye such as a pupil (e.g. a bright pupil 702) and an iris 604. In examples, an advantage of using a second IR LED 107 to create a bright pupil effect may be to improve the accuracy of a gaze estimation system 300 in locating a pupil region in an image. As shown in FIG. 7C, one or more corneal reflections 124 on the corneal surface of the user's eye may also be visible in the IR camera image 302.

FIG. 8 illustrates an example embodiment of the eye-tracking system 100, where the eye-tracking system 100 is located within a head mounted device (HMD) 910, in accordance with examples of the present disclosure. In the present embodiment, a user 600 interacts with the HMD eye-tracking system by gazing at a display screen 108 within the HMD 910 through a convex lens 802. A gaze vector 120 represents the direction of the user's gaze. In the present embodiment, a first IR LED 106a, a second IR LED 106b and an IR camera 104 are located in proximity to the user's eyes. In examples, the first IR LED 106a and the second IR LED 106b may each generate a corneal reflection 124. In the present embodiment, for example, due to the close proximity of the hardware of the eye-tracking system 100, it may be necessary to use two IR LEDs for illumination of the user's eyes, to ensure even illumination in the captured IR camera image 302 and to ensure at least one corneal reflection 124 is captured in the IR camera image 302 across a full range of eye movements and positions. FIG.s 8B-D illustrate example IR camera images 302 obtained for the embodiment of FIG 8A over a range of eye positions. FIG. 8B shows an example IR camera image 302 where the user's eye is gazing forward and two corneal reflections 124a and 124b are visible in the IR camera image 302, each corneal reflection corresponding to a respective IR LED. FIG 8C shows an example IR camera image 302 where the user's eye is gazing far to the right, however only one corneal reflection 124 is captured. A secondary reflection (e.g. a sclera reflection 804) is visible on the sclera of the user's eye. Similarly, FIG 8D shows an example IR camera image 302 where the user's eye is gazing far to the left, however only one corneal reflection 124 is captured while a secondary sclera reflection 804 is also visible.

FIG. 9 is a block diagram illustrating an example architecture of the gaze estimation system 300 that may be used to implement methods to estimate a gaze vector 120 representing a gaze direction in conjunction with the embodiment of FIG 8A, in accordance with example embodiments of the present disclosure.

In some examples, the gaze estimation system 300 receives an IR camera image 302 (e.g. an IR eye image 306) and outputs an estimated gaze vector 120 including gaze angles, where the gaze vector 120 represents a gaze direction of a user. The IR camera image 302 may be captured by the IR camera 104 on the computing system 200 or the IR camera image 302 may be a digital image taken by another IR camera on another electronic device and communicated to the computing system 200 (e.g., in the case where the computing system 200 provides a gaze estimation service to other devices). In examples, the IR camera image 302 may be extracted from video images captured by the IR camera 104. In the present embodiment, the IR camera image 302 may be an IR eye image 306 obtained in close proximity to a user by an IR camera 104 within a head mounted device (HMD) 910, where the IR eye image 306 is an IR image encompassing a user's eye.

In some embodiments, for example, the HMD 910 of the gaze estimation system 300 may output positional information for the user's head as a distance of eye from camera 920. In examples, a HMD 910 includes an adjustable headset worn by a user, where the IR camera 104 is fixed within the headset and a distance from the IR camera 104 to the user's eye may be determined based on physical information provided by the HMD 910. In examples, when a user adjusts the headset of the HMD 910, measurements may be obtained by the HMD 910 corresponding to the user adjustments or fit settings and used to determine the distance of eye from camera 920. Optionally, a pupillary distance (PD) (e.g. the distance measured in millimeters between the 2D pupil centers of the eyes) may also be obtained by the HMD 910 and used to determine the distance of eye from camera 920.

In examples, the IR eye image 306 may be input to a corneal reflection estimator 330 to obtain at least one position of a corneal reflection 124 in the IR eye image 306, where a corneal reflection 124 is a virtual image of the reflection of a respective IR LED 106 on the cornea surface of the user's eye, and where the number of corneal reflections 124 depends on the number of IR LEDs 106. An example of a corneal reflection estimator 330 that can be implemented in example embodiments is described in: Chugh, S. et al. "Detection and Correspondence Matching of Corneal Reflections for Eye Tracking Using Deep Learning" in Proceedings of the 25th International Conference on Pattern Recognition (ICPR), IEEE, 2020.

In some embodiments, the position of at least one corneal reflection 124 may be input to a cornea center estimator 340. In examples, the cornea center estimator 340 may also receive positional information as the distance of eye from camera 920 and may output a position of a cornea center $c$ 112, for example, as 3D coordinates $(x,y,z)$, based on the at least one corneal reflection 124 and the positional information. In examples, the cornea center estimator 340 may be a block that computes the position of the cornea center $c$ 112 based on equations 2-5 previously described with respect to FIG. 3, where $k_{qi}$ represents the distance of eye from camera 920.

In some embodiments, for example, the IR eye image 306 and the position of the cornea center 112 may be input to a pupil center estimator 350 to generate a position of a 3D pupil center 114. An example of a pupil center estimator 350 that can be implemented in example embodiments is described in: Guestrin, E. D., & Eizenman, M. (2006). General theory of remote gaze estimation using the pupil center and corneal reflections. IEEE Transactions on biomedical engineering, 53(6), 1124-1133.

In some embodiments, for example, the cornea center 112 and the 3D pupil center 114 may be input to a gaze estimator 360 along with subject-specific calibration parameters 520 to generate a gaze vector 120, as described previously in the discussion of FIG. 5.

FIG. 10 is a flowchart illustrating steps of an example method 1000 for estimating a gaze vector 120 representative of a user's gaze direction, in accordance with examples of the present disclosure. The method 1000 can be performed by the computing system 200. For example, the processor 202 can execute computer readable instructions (which can be stored in the memory 214) to cause the computing system 200 to perform the method 1000. The method 1000 may be performed using a single physical machine (e.g., a workstation or server), a plurality of physical machines working together (e.g., a server cluster), or cloud-based resources (e.g., using virtual resources on a cloud computing platform).

Method 1000 begins with step 1002 in which an IR camera image 302 of a user is obtained using an IR camera 104 and at least one IR LED 106, where the IR camera image 302 includes an image of an eye of the user. The IR camera image 302 may be captured by an IR camera 104 on the computing system 100 or may be a digital image taken by another IR camera on another electronic device and communicated to the computing system 100.

At step 1004, a position of a corneal reflection 124 is estimated within the IR camera image 302, using a corneal reflection estimator. In examples, the corneal reflection 124 is a virtual image of the reflection of a respective IR LED 106 on the cornea surface of the user's eye captured in the IR camera image 302.

At step 1006, a positional information for the user's head may be obtained. In some embodiments, a positional information for the user's head may be a head pose 315 or in other embodiments a positional information for the user's head may be a distance of eye from camera 920.

At step 1008, a position of a cornea center 112 for the user's eye is estimated using a cornea center estimator 340, based on the position of the corneal reflection 124 and the positional information.

At step 1010 a position of a 3D pupil center 114 is estimated using a pupil center estimator 350, based on the IR camera image 302 and the position of the cornea center 112.

Finally, at step 1012 a gaze vector 120, representing a gaze direction may be estimated using a gaze estimator 360, based on the position of the cornea center 112 and the position of the 3D pupil center 114. The estimated gaze vector 120 may contain two angles describing the gaze direction, the angles being a yaw and a pitch.

In some examples, the estimated gaze direction may be output to an application on an electronic device (e.g., a software application executed by the computing system 200) to estimate the point on the screen of the electronic device that an individual is looking at. For example, if the application on the electronic device is an assistive tool to enable speech generation, obtaining accurate estimates of a point of gaze on a screen may enable a non-verbal individual to communicate by gazing at specific areas of the screen to spell words or assemble sentences. In another example, if the application on the electronic device is an educational application, gathering data on where and how long users look at certain areas of the screen can provide feedback to the provider of the educational application on the effectiveness of the educational content, what content holds the user's attention and what content is missed. Similarly, if the application on the electronic device contains advertising or marketing content, data can be gathered on the effectiveness of the content by examining if and for how long an individual looks at an advertisement. Data may be gathered to understand optimal placement of content on the screen or identify effective content that attracts an individual's attention more often and holds their attention for longer.

In other examples, the estimated gaze direction may be output to an application to be executed by an invehicle computing system to assess the point of gaze of an individual operating the vehicle. In situations where the individual operating the vehicle appears to be distracted or inattentive, for example, looking away from the road frequently or for extended periods, the vehicle safety system may provide a notification or an alert to the operator of the vehicle to remind them to pay attention to the road ahead.

Although the present disclosure describes methods and processes with steps in a certain order, one or more steps of the methods and processes may be omitted or altered as appropriate. One or more steps may take place in an order other than that in which they are described, as appropriate.

Although the present disclosure is described, at least in part, in terms of methods, a person of ordinary skill in the art will understand that the present disclosure is also directed to the various components for performing at least some of the aspects and features of the described methods, be it by way of hardware components, software or any combination of the two. Accordingly, the technical solution of the present disclosure may be embodied in the form of a software product. A suitable software product may be stored in a pre-recorded storage device or other similar non-volatile or non-transitory computer readable medium, including DVDs, CD-ROMs, USB flash disk, a removable hard disk, or other storage media, for example. The software product includes instructions tangibly stored thereon that enable a processing device (e.g., a personal computer, a server, or a network device) to execute examples of the methods disclosed herein. The machine-executable instructions may be in the form of code sequences, configuration information, or other data, which, when executed, cause a machine (e.g., a processor or other processing device) to perform steps in a method according to examples of the present disclosure.

The present disclosure may be embodied in other specific forms without departing from the subject matter of the claims. The described example embodiments are to be considered in all respects as being only illustrative and not restrictive. Selected features from one or more of the above-described embodiments may be combined to create alternative embodiments not explicitly described, features suitable for such combinations being understood within the scope of this disclosure. All values and sub-ranges within disclosed ranges are also disclosed. Also, although the systems, devices and processes disclosed and shown herein may comprise a specific number of elements/components, the systems, devices and assemblies could be modified to include additional or fewer of such elements/components. For example, although any of the elements/components disclosed may be referenced as being singular, the embodiments disclosed herein could be modified to include a plurality of such elements/components. The subject matter described herein intends to cover and embrace all suitable changes in technology.

**Claims**

1. An eye-tracking system comprising:

one or more processors; and
a memory storing machine-executable instructions which, when executed by the processor device, cause the system to:

obtain an image of a user, the image including an image of an eye of the user;

estimate a position of a corneal reflection in the image;
determine a positional information for the user's head based on the image; and

estimate a position of a cornea center for the user's eye based on the position of the corneal reflection in the image and the positional information,

wherein the system further comprises an IR camera and a first IR LED;
wherein the image of a user is obtained by using the IR camera and the first IR LED,
wherein machine-executable instructions, when executed by the one or more processors to estimate the position of the cornea center for the user's eye, further cause the system to:
determine a distance between the user's eye and the IR camera based on the positional information; and estimate the position of the cornea center based on the distance between the user's eye and the IR camera, a radius of the user's cornea, a location of the IR camera, a location of the at least one IR LED and the position of the corneal reflection in the image, wherein the system further comprises a second IR LED, wherein the first IR LED generates the corneal reflection in the image and the second IR LED generates a bright pupil in the image.

2. The system of claim 1, wherein the machine-executable instructions, when executed by the one or more processors, further cause the system to:
estimate a position of a 2D pupil center in the image;

estimate a position of a 3D pupil center for the user's eye based on position of the 2D pupil center in the image and the position of the cornea center; and
estimate a gaze vector representing the user's gaze direction based on the position of the cornea center and the position of the 3D pupil center.

3. The system of claim 2, wherein the machine-executable instructions, when executed by the one or more processors to estimate the gaze vector representing the user's gaze direction, further cause the system to:

estimate an optical axis of the user's eye based on the position of the cornea center and the position of the 3D pupil center; and
estimate the gaze vector based on the optical axis and a plurality of calibration parameters.

4. The system of any one of claims 1 to 3, wherein the positional information for the user's head includes a head pose of the user.

5. The system of claim 4, wherein the machine-executable instructions, when executed by the one or more processors to obtain the head pose of the user, further cause the system to:

estimate one or more face landmarks corresponding to a face of the user, based on the image;
fit the estimated one or more face landmarks to a 3D face model; and

estimate a head pose based on the 3D face model.

6. The system of any one of claims 1 to 5, wherein the IR camera is positioned at a distance from the user and the image is a face image.

7. The system of any one of claims 1 to 3, wherein the IR camera image is an eye image and the positional information for a user's head is a distance of a user's eye from the IR camera, the distance of the user's eye from the IR camera being obtained from a head mounted device.

**Patentansprüche**

1. Blickverfolgungssystem, umfassend:

einen oder mehrere Prozessoren; und
einen Speicher, der maschinenausführbare Anweisungen speichert, die, wenn sie durch den Prozessor ausgeführt werden, das System zu Folgendem veranlassen:

Erlangen eines Bildes eines Benutzers, wobei das Bild ein Bild eines Auges des Benutzers beinhaltet;
Schätzen einer Position einer Hornhautreflexion in dem Bild;
Bestimmen von Positionsinformationen für den Kopf des Benutzers basierend auf dem Bild; und
Schätzen einer Position eines Hornhautzentrums für das Auge des Benutzers basierend auf der Position der Hornhautreflexion in dem Bild und den Positionsinformationen, wobei das System ferner eine IR-Kamera und eine erste IR-LED umfasst;

wobei das Bild eines Benutzers unter Verwendung der IR-Kamera und der ersten IR-LED erlangt wird,
wobei maschinenausführbare Anweisungen, wenn sie durch einen oder mehrere Prozessoren ausgeführt werden, um die Position des Hornhautzentrums für das Auge des Benutzers zu schätzen, das System ferner zu Folgendem veranlassen:

Bestimmen eines Abstands zwischen dem Auge des Benutzers und der IR-Kamera basierend auf den Positionsinformationen; und
Schätzen der Position des Hornhautzentrums basierend auf dem Abstand zwischen dem Auge des Benutzers und der IR-Kamera, einem Radius der Hornhaut des Benutzers, einem Standort der IR-Kamera, einem Standort der mindestens einen IR-LED und der Position der Hornhautreflexion in dem Bild,

wobei das System ferner eine zweite IR-LED umfasst, wobei die erste IR-LED die Hornhautreflexion in dem Bild erzeugt und die zweite IR-LED eine helle Pupille in dem Bild erzeugt.

2. System nach Anspruch 1, wobei die maschinenausführbaren Anweisungen, wenn sie durch den einen oder die mehreren Prozessoren ausgeführt werden, das System ferner zu Folgendem veranlassen:

Schätzen einer Position eines 2D-Pupillenzentrums in dem Bild;
Schätzen einer Position eines 3D-Pupillenzentrums für das Auge des Benutzers basierend auf der Position des 2D-Pupillenzentrums in dem Bild und der Position des Hornhautzentrums; und
Schätzen eines Blickvektors, der die Blickrichtung des Benutzers darstellt, basierend auf der Position des Hornhautzentrums und der Position des 3D-Pupillenzentrums.

3. System nach Anspruch 2, wobei die maschinenausführbaren Anweisungen, wenn sie durch den einen oder die mehreren Prozessoren ausgeführt werden, um den Blickvektor zu schätzen, der die Blickrichtung des Benutzers darstellt, das System ferner zu Folgendem veranlassen:

Schätzen einer optischen Achse des Auges des Benutzers basierend auf der Position des Hornhautzentrums und der Position des 3D-Pupillenzentrums; und
Schätzen des Blickvektors basierend auf der optischen Achse und einer Vielzahl von Kalibrierungsparametern.

4. System nach einem der Ansprüche 1 bis 3, wobei die Positionsinformationen für den Kopf des Benutzers eine Kopfhaltung des Benutzers beinhalten.

5. System nach Anspruch 4, wobei die maschinenausführbaren Anweisungen, wenn sie durch den einen oder die mehreren Prozessoren ausgeführt werden, um die Kopfhaltung des Benutzers zu erlangen, das System ferner zu Folgendem veranlassen:

Schätzen, basierend auf dem Bild, eines oder mehrerer Gesichtsmerkmale, die einem Gesicht des Benutzers entsprechen; Abgleichen des einen oder der mehreren geschätzten Gesichtsmerkmale mit einem 3D-Gesichtsmodell; und Schätzen einer Kopfhaltung basierend auf dem 3D-Gesichtsmodell.

6. System nach einem der Ansprüche 1 bis 5, wobei die IR-Kamera in einem Abstand von dem Benutzer positioniert ist und es sich bei dem Bild um ein Gesichtsbild handelt.

7. System nach einem der Ansprüche 1 bis 3, wobei es sich bei dem Bild der IR-Kamera um ein Augenbild handelt und die Positionsinformationen für den Kopf des Benutzers ein Abstand eines Auges des Benutzers von der IR-Kamera sind, wobei der Abstand des Auges des Benutzers von der IR-Kamera von einer an einem Kopf montierten Vorrichtung erlangt wird.

**Revendications**

1. Système de suivi oculaire comprenant :

un ou plusieurs processeurs ; et
une mémoire stockant des instructions exécutables par machine qui, lorsqu'elles sont exécutées par le dispositif de processeur, amènent le système à :

obtenir une image d'un utilisateur, l'image comportant une image d'un œil de l'utilisateur ;
estimer la position d'une réflexion cornéenne dans l'image ;
déterminer des informations de position de la tête de l'utilisateur sur la base de l'image ; et
estimer la position du centre de la cornée de l'œil de l'utilisateur sur la base de la position de la réflexion cornéenne dans l'image et des informations de position,
dans lequel le système comprend également une caméra IR et une première DEL IR ;
dans lequel l'image d'un utilisateur est obtenue en utilisant la caméra IR et la première DEL IR,

dans lequel les instructions exécutables par machine, lorsqu'elles sont exécutées par les un ou plusieurs processeurs pour estimer la position du centre de la cornée de l'œil de l'utilisateur, amènent également le système à :

déterminer la distance entre l'œil de l'utilisateur et la caméra IR sur la base des informations de position ; et estimer la position du centre de la cornée sur la base de la distance entre l'œil de l'utilisateur et la caméra IR, du rayon de la cornée de l'utilisateur, de la position de la caméra IR, de la position de l'au moins une DEL IR et de la position de la réflexion cornéenne dans l'image, dans lequel le système comprend également une seconde DEL IR, dans lequel la première DEL IR génère la réflexion cornéenne dans l'image et la seconde DEL IR génère une pupille brillante dans l'image.

2. Système selon la revendication 1, dans lequel les instructions exécutables par machine, lorsqu'elles sont exécutées par les un ou plusieurs processeurs, amènent également le système à :

estimer la position du centre d'une pupille 2D dans l'image ; estimer la position du centre d'une pupille 3D pour l'œil de l'utilisateur sur la base de la position du centre de la pupille 2D dans l'image et de la position du centre de la cornée ; et estimer un vecteur de regard représentant la direction du regard de l'utilisateur sur la base de la position du centre de la cornée et de la position du centre de la pupille 3D.

3. Système selon la revendication 2, dans lequel les instructions exécutables par machine, lorsqu'elles sont exécutées par les un ou plusieurs processeurs pour estimer le vecteur de regard représentant la direction du regard de l'utilisateur, amènent également le système à :

estimer l'axe optique de l'œil de l'utilisateur sur la base de la position du centre de la cornée et de la position du centre de la pupille 3D ; et estimer le vecteur de regard sur la base de l'axe optique et d'une pluralité de paramètres d'étalonnage.

4. Système selon l'une quelconque des revendications 1 à 3, dans lequel les informations de position de la tête de l'utilisateur comportent une pose de tête de l'utilisateur.

5. Système selon la revendication 4, dans lequel les instructions exécutables par machine, lorsqu'elles sont exécutées par les un ou plusieurs processeurs pour obtenir la pose de tête de l'utilisateur, amènent également le système à :

estimer un ou plusieurs points de repère faciaux correspondant au visage de l'utilisateur, sur la base de l'image ; adapter les un ou plusieurs points de repère faciaux estimés à un modèle facial 3D ; et estimer la pose de tête sur la base du modèle facial 3D.

6. Système selon l'une quelconque des revendications 1 à 5, dans lequel la caméra IR est positionnée à une distance de l'utilisateur et l'image est une image de visage.

7. Système selon l'une quelconque des revendications 1 à 3, dans lequel l'image de la caméra IR est une image de l'œil et les informations de position de la tête d'un utilisateur sont une distance entre l'œil de l'utilisateur et la caméra IR, la distance entre l'œil de l'utilisateur et la caméra IR étant obtenue à partir d'un dispositif monté sur la tête.

**FIG. 1**

EP 4 572 655 B1

Computing system 200

206 Input device

104 IR Camera

210 Output device

202 Processor

204 I/O interface

212 Communications interface

214 Memory

216 Data

300-I Instructions

302 IR Camera Image

520 Calibration Parameters

**FIG. 2**

**FIG. 3**

Gaze Estimation System 300

304 IR Face Image

310 Head Pose Estimator

315 Head Pose

340 Cornea Center Estimator

112 Cornea Center

124 2D Corneal Reflection

320 Eye Region Extractor

325 Eye Region Image

330 Corneal Reflection Estimator

350 Pupil Center Estimator

114 3D Pupil Center

360 Gaze Estimator

120 Gaze Vector

EP 4 572 655 B1

18

EP 4 572 655 B1

**FIG. 4**

**FIG. 5**

**FIG. 6A**

**FIG. 7A**

FIG. 6C

FIG. 7C

FIG. 6B

FIG. 7B

FIG. 8B

FIG. 8C

FIG. 8D

FIG. 8A

**FIG. 9**

1000

1002 Obtain an IR camera image of a user using an IR camera and at least one IR LED, the IR camera image including an image of an eye of the user

1004 Estimate a position of a corneal reflection based on the IR camera image

1006 Determine a positional information for the user's head based on the IR camera image

1008 Estimate a position of a cornea center for the user's eye based on the position of the corneal reflection and the positional information

1010 Estimate a position of a 3D pupil center based on the IR camera image and the position of the cornea center

1012 Estimate a gaze vector representing the user's gaze direction based on the position of the cornea center and the position of the 3D pupil center

# FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019213402 A1

**Non-patent literature cited in the description**

- **WU** ; **ZHENGYANG et al.** Magiceyes: A large scale eye gaze estimation dataset for mixed reality. *arXiv preprint arXiv: 2003.08806*, 2020
- **ZHU** ; **ZHIWEI** ; **QIANG JI**. Novel eye gaze tracking techniques under natural head movement. *IEEE Transactions on biomedical engineering*, 2007, vol. 54 (12), 2246-2260
- A Novel Cross-Ratio Based Gaze Estimation Method. **FAN** ; **SHUO** ; **JIANNAN CHI** ; **JIAHUI LIU**. 2022 International Conference on Machine Learning and Knowledge Engineering (MLKE). IEEE, 2022
- **GUESTRIN, E. D** ; **EIZENMAN, M.** General theory of remote gaze estimation using the pupil center and corneal reflections. *IEEE Transactions on biomedical engineering*, 2006, vol. 53 (6), 1124-1133
- **GUESTRIN, E. D** ; **EIZENMAN, M**. General theory of remote gaze estimation using the pupil center and corneal reflections. *IEEE Transactions on biomedical engineering*, 2006, vol. 53 (6), 1124-1133
- Detection and Correspondence Matching of Corneal Reflections for Eye Tracking Using Deep Learning. **CHUGH, S. et al.** Proceedings of the 25th International Conference on Pattern Recognition (ICPR. IEEE, 2020
- **GUESTRIN, E. D.** ; **EIZENMAN, M**. General theory of remote gaze estimation using the pupil center and corneal reflections. *IEEE Transactions on biomedical engineering*, 2006, vol. 53 (6), 1124-1133
- Detection and Correspondence Matching of Corneal Reflections for Eye Tracking Using Deep Learning. **CHUGH, S. et al.** Proceedings of the 25th International Conference on Pattern Recognition (ICPR). IEEE, 2020
- **GUESTRIN, E. D** ; **EIZENMAN, M**. General theory of remote gaze estimation using the pupil center and corneal reflections.. *IEEE Transactions on biomedical engineering*, 2006, vol. 53 (6), 1124-1133